# EUROPEAN PATENT APPLICATION

(11) **EP 4 570 901 A1**
(43) Date of publication of application: **18.06.2025**
(21) Application number: 24217822.6
(22) Date of filing: 05.12.2024
(51) Int. Cl.: C12N 5/00, C12N 5/0793, C12N 5/074, A61K 35/30

(54) **METHOD FOR SELECTIVE REMOVAL OF UNDIFFERENTIATED PLURIPOTENT STEM CELLS**

(30) Priority: 12.12.2023 TW 112148328
(71) Applicant: Buddhist Tzu Chi Medical Foundation, Hualien 970 (TW)
(72) Inventor: CHANG, Chia-Yu, Hualien (TW); TING, Hsiao-Chien, Hualien (TW); LIN, Shinn-Zong, Hualien (TW); HARN, Horng-Jyh, Hualien (TW); LIU, Ching-Ann, Hualien (TW); SU, Hong-Lin, Taichung City (TW); CHIOU, Tzyy-Wen, Hualien (TW); CHEN, Yu-Shuan, Hualien (TW)
(74) Representative: Lewis Silkin LLP

(57) **Abstract**

The present disclosure provides a method for selective removal of undifferentiated pluripotent stem cells including inducing stem cells differentiation, and then administering an effective amount of phthalide for the removal of undifferentiated pluripotent stem cells.

## Description

### TECHNICAL FIELD

The present disclosure relates to a method for culturing stem cells, particularly a method for selectively removing undifferentiated stem cells.

### BACKGROUND

Stem cells are cells with the capacity for self-renewal and differentiation. Based on differentiation potential, stem cells are generally categorized from highest to lowest into totipotent stem cells, pluripotent stem cells, oligopotent stem cells, and unipotent stem cells.

Pluripotent stem cells refer to undifferentiated cells that have the ability to self-renew and can differentiate into cells from the three germ layers of the human body. These pluripotent stem cells can be classified into embryonic stem cells and induced pluripotent stem cells based on their origin. Embryonic stem cells are derived from the inner cell mass of human blastocyst-stage embryos and can differentiate into various types of somatic cells. Induced pluripotent stem cells, which possess similar properties and differentiation potential as embryonic stem cells, can be generated by reprogramming somatic cells through the delivery of specific genes and proteins or the use of small chemical molecules.

Pluripotent stem cells have the capacity to differentiate into a variety of somatic cells, can be cultured and expanded in large quantities *in vitro,* and can be induced to form specific somatic cells, organs, and tissues. The *in vitro* differentiation process of pluripotent stem cells can simulate embryonic development, enhancing our understanding of human developmental processes. In disease research, pluripotent stem cells derived from patients can be transformed into various types of pathological cells, aiding in the exploration of unknown disease processes and pathogenic mechanisms, as well as in drug development and precision medicine.

Compared to pluripotent stem cells, oligopotent stem cells have a lower differentiation potential but still retain the ability to differentiate into somatic cells. Oligopotent stem cells can be isolated from adults, with common types including mesenchymal stem cells derived from umbilical cord, umbilical cord blood, placenta, amniotic fluid, bone marrow, and adipose tissue.

Many researchers focused on applying pluripotent and oligopotent stem cells in the cell transplantation treatment of various diseases. It is anticipated that differentiated cells, such as neural cells, glial cells, ocular cells, cardiomyocytes, blood cells, islet cells, and mesenchymal stem cells, can be used in the treatment of Parkinson's disease, spinal cord injury, macular degeneration, corneal transplantation, myocardial infarction, tumor immunotherapy, diabetes, and autoimmune diseases, with several of these applications already in clinical trials.

During the differentiation process of specific somatic cells for transplantation, if any residual undifferentiated pluripotent stem cells remain, there is a risk of teratoma formation after transplantation into a living organism, which increases the potential for cancer. Current methods for removing undifferentiated pluripotent stem cells from transplanted cell populations primarily involve flow cytometry sorting to exclude cells with pluripotent stem cell-specific surface antigens or selecting cells with specific somatic cell surface antigens to avoid the presence of undifferentiated pluripotent stem cells. Although this method can reduce the residual undifferentiated pluripotent stem cells, it is time-consuming, costly, and labor-intensive, and the genetic, cellular properties, and health of certain specific somatic cells may be altered after sorting. Recent studies have reported that early treatment with quercetin can reduce the presence of residual undifferentiated pluripotent stem cells.

In summary, to reduce the risks associated with the use of stem cells in clinical cell transplantation treatments and to improve the safety of the applications of stem cells, there is an urgent need to develop methods for selectively removing undifferentiated pluripotent stem cells, which is a crucial and essential point for reducing the carcinogenic potential of undifferentiated pluripotent stem cells.

### SUMMARY

The present disclosure relates to a method for selectively removing undifferentiated pluripotent stem cells. The method includes: collecting pluripotent stem cells; inducing differentiation of the collected pluripotent stem cells to obtain a cell population comprising differentiated cells and undifferentiated pluripotent stem cells; and administering an effective amount of a phthalide compound to the cell population to selectively remove the undifferentiated collected stem cells in the cell population.

In one aspect of the present disclosure, the phthalide compound is selected from the group consisting of n-butylidenephthalide, methyl phthalide, 7-methyl phthalide, ethyl phthalide, n-propenyl phthalide, n-butyl phthalide, 3-bromophthalide, 5-bromophthalide, 5-chlorophthalide, 6-chlorophthalide, 3,4-dichlorophthalide, tetrachlorophthalide, 3-hydroxy-3-trifluoromethylphthalide, 3-methyl-3-(1-naphthyl)phthalide, 3-(5-fluoro-1-naphthyl)phthalide, 4-amino-3-hydroxyphthalide, 5-carboxyphthalide, 5-cyanophthalide, 7-methoxyphthalide, 7-hydroxy-6-methoxyphthalide, 3-(1,2-dimethyl-3-indolyl)phthalide, phenolphthalein, ligustilide, and serdanolide. In another aspect of the present disclosure, the phthalide compound include n-butylidenephthalide, n-butyl phthalide, tetrachlorophthalide, and phenolphthalein. In yet another aspect, the phthalide compound is n-butylidenephthalide.

In one aspect of the present disclosure, the effective amount of the phthalide compound is administered during the differentiation of the pluripotent stem cells. During the differentiation period, the differentiated cells include at least one selected from the group consisting of oligopotent stem cells, unipotent stem cells, and somatic cells. In one aspect, the differentiated cells in the cell population do not include somatic cells. In another aspect, the effective amount of the phthalide compound is administered after somatic cells are present in the cell population.

In one aspect of the present disclosure, an concentration of the effective amount of the phthalide compound is from 10 µM to 1000 µM. In another aspect, the concentration of the effective amount of the phthalide compound is from 50 µM to 800 µM.

In one aspect of the present disclosure, the effective amount of the phthalide compound is administered for 1 to 6 days.

In one aspect of the present disclosure, the pluripotent stem cells are embryonic stem cells or induced pluripotent stem cells.

The present disclosure effectively reduces the risk associated with the subsequent use of differentiated pluripotent stem cells and improves the safety of their applications by selectively removing undifferentiated pluripotent stem cells from a population of induced differentiated pluripotent stem cells while retaining the differentiated cells through treatment with an effective amount of the phthalide compound.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Figures 1A and 1B are cell morphology diagrams of human induced pluripotent stem cells (iPSCs) treated with different concentrations of n-butylidenephthalide.
Figure 2 shows the cell morphology of embryoid bodies derived from human iPSCs treated with different concentrations of n-butylidenephthalide.
Figures 3A and 3B are cell morphology diagrams of human iPSCs treated with different concentrations of n-butylidenephthalide after differentiation into neural stem cells.
Figure 4A is an immunofluorescence staining diagram illustrating the degree of differentiation of human iPSCs differentiated by the SFEB suspension method on day 25. The cells have differentiated into neurons (i.e., neuronal cells). Figures 4B and 4C are cell morphology diagrams of human iPSCs treated with different concentrations of n-butylidenephthalide after differentiation into neuronal cells.

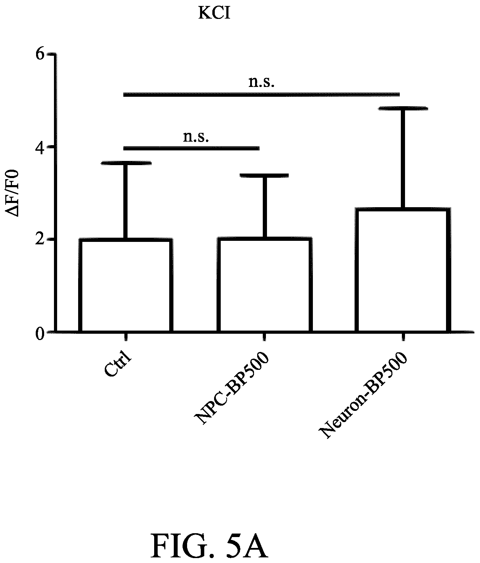

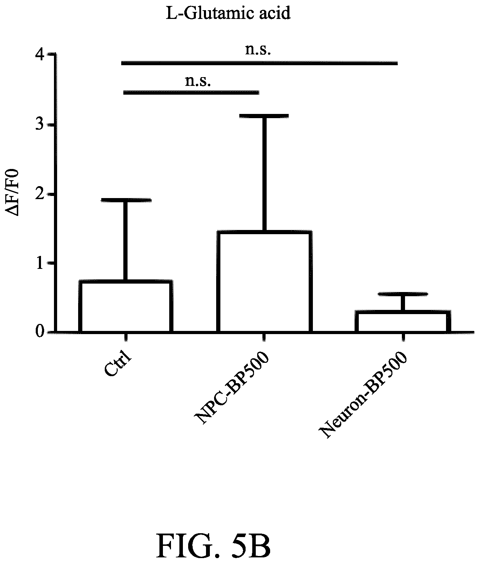

Figures 5A and 5B are statistical diagrams showing changes in calcium ion levels in differentiated neurons in response to neurotransmitters after treatment with n-butylidenephthalide. The groups include: (1) cells cultured without n-butylidenephthalide treatment (Ctrl) responding to neurotransmitters; (2) cells cultured with 500 µM n-butylidenephthalide for 120 hours on day 10 of differentiation (NPC-BP500) responding to neurotransmitters; and (3) cells cultured on day 25 of differentiation with 500 µM n-butylidenephthalide for 120 hours (Neuron-BP500) responding to neurotransmitters. Statistical analysis showed no significant differences in calcium ion changes in neurons after stimulation with KCl and glutamic acid among the groups. All tests were conducted on the 38th day (d38) of differentiation.
Figure 6 is an immunofluorescence staining image showing the expression of Oct-4 and N-cadherin (Ncad) in differentiated neural cells treated with n-butylidenephthalide.
Figure 7 is a flow chart illustrating the procedure for testing the identification of differentiating dopamine neurons.

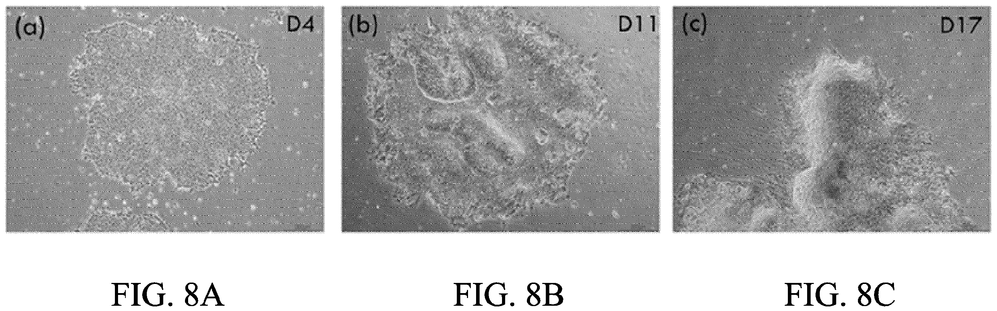

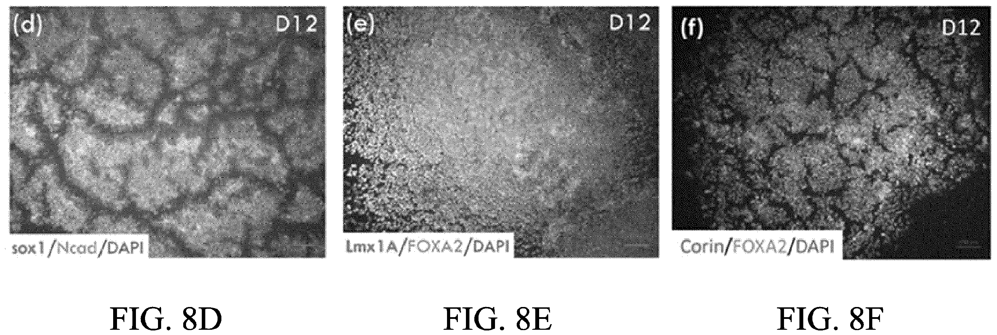

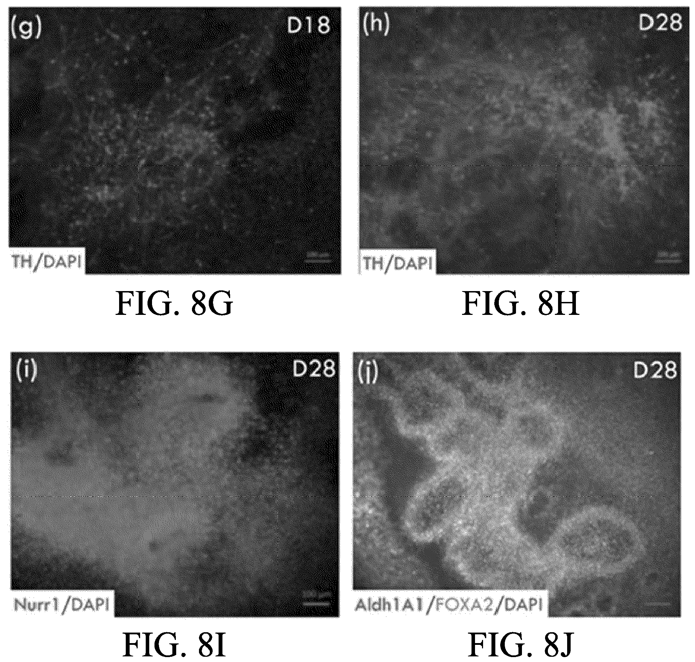

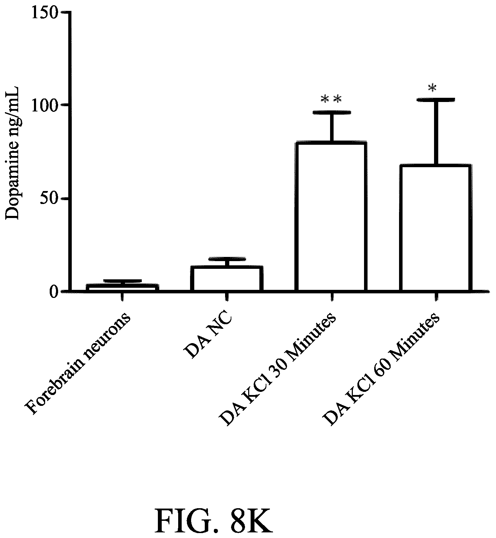

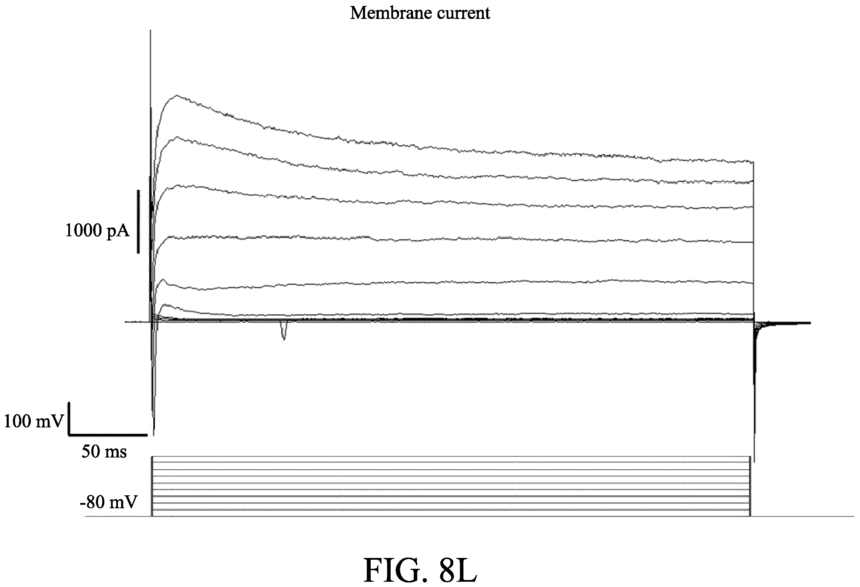

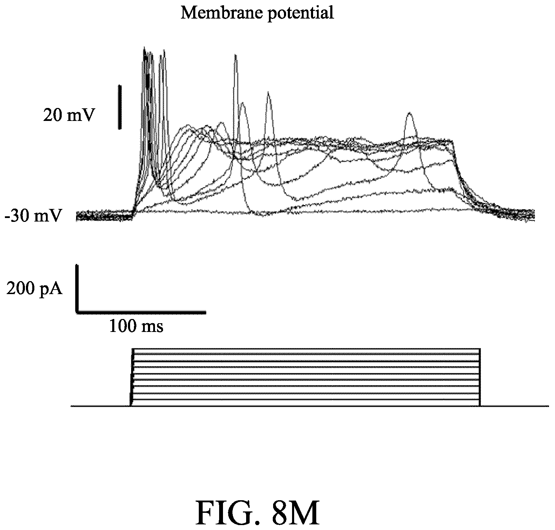

Figures 8A to 8M illustrate the test results for the identification of differentiating dopamine neurons. Figures 8A to 8C show cell morphology at different stages of differentiation; Figures 8D to 8F are immunofluorescence staining diagrams identifying specific proteins in neural stem cells and dopamine precursor cells; Figures 8G to 8J display immunofluorescence staining of differentiated cells using dopamine neuron-specific antigens on days 18 to 28 of differentiation; Figure 8K is a bar graph showing dopamine secretion ability; Figures 8L and 8M present membrane current and membrane potential graphs for testing neural electrophysiological function.
Figures 9A and 9B are diagrams showing, respectively, a motor ability test and brain tissue immunofluorescence staining of Parkinson's disease model rats transplanted with dopamine neurons treated with n-butylidenephthalide.
Figure 10 is a survival rate comparison graph between Parkinson's disease model rats transplanted with dopamine neurons (preBP-DA) and the control group (Medium).

### DETAILED DESCRIPTION OF THE EMBODIMENTS

The technical solutions set forth in the embodiments of the present disclosure will be described more clearly and completely below. It is apparent that the described embodiments are only some of the many possible embodiments encompassed by the present disclosure and are not intended to limit the scope of the present disclosure. The present disclosure may also be practiced or used in similar or different embodiments. Modifications, changes, substitutions of certain elements or combinations thereof, and other implementations that can be made by those skilled in the art without creative effort are all included within the scope of the present disclosure.

Further, it should be noted that the singular forms "a," "an," and "the," as used herein, are intended to include plural referents unless explicitly limited to a single referent. Additionally, the term "or" is used interchangeably with the term "and/or" unless the context clearly indicates otherwise.

As used herein, the term "about" refers to a variance or range within 20%, preferably within 10%, and more preferably within 5% of the stated numerical value, numerical range, or ratio. The quantitative values stated herein are approximations and are intended to be inferred even when the term "about" is not explicitly used. The numerical ranges stated herein encompass all values within those ranges. For example, the range of 50 µM to 800 µM includes values such as 50 µM, 50.01 µM, 50.1 µM, and 50.5 µM, and also includes all sub-ranges within the stated range, such as 50 µM to 700 µM, 88 µM to 650 µM, and 166 µM to 521 µM.

The terms "including," "comprising," "containing," and "having," as used herein, refer to the inclusion of specified elements (such as components or steps) in the objects, methods, or uses of the present disclosure. Unless the context clearly indicates otherwise, these terms are intended to be open-ended, meaning that unspecified elements may also be present in the objects, methods, and uses of the present disclosure, whether or not they are necessary. Therefore, the exclusion of unspecified elements is not implied by these terms.

The term "stem cells," as used herein, refers to cells with the potential for self-renewal and differentiation into somatic cells, including totipotent stem cells, pluripotent stem cells, oligopotent stem cells, and unipotent stem cells. Stem cells may be isolated from a cell population containing stem cells (such as embryonic stem cells or mesenchymal stem cells) or produced by the reprogramming of somatic cells (such as induced pluripotent stem cells).

The term "removal of undifferentiated collected pluripotent stem cells," as used herein, refers to the removal of undifferentiated pluripotent stem cells from a cell population after at least a portion of the collected pluripotent stem cells have differentiated into oligopotent stem cells, unipotent stem cells, and/or somatic cells. In one embodiment of the present disclosure, the pluripotent stem cells may be, but are not limited to, embryonic stem cells or induced pluripotent stem cells; the oligopotent stem cells may be, but are not limited to, neural stem cells, mesenchymal stem cells, or hematopoietic stem cells.

The term "effective amount," as used herein, refers to the dosage of a compound that produces the desired effect, specifically, the dosage of a phthalide compound that results in the selective removal of undifferentiated pluripotent stem cells.

The present disclosure relates to a method for selectively removing undifferentiated pluripotent stem cells, comprising: collecting pluripotent stem cells; inducing differentiation of the collected pluripotent stem cells to obtain a cell population comprising differentiated cells, wherein the cell population further comprises undifferentiated collected pluripotent stem cells; and administering an effective amount of a phthalide compound to the cell population to selectively remove the undifferentiated collected pluripotent stem cells.

In some embodiments of the present disclosure, the method involves culturing and subculturing the pluripotent stem cells before collection.

Methods for inducing the differentiation of pluripotent stem cells are well known to those skilled in the art. A non-limiting example of methods for inducing differentiation of pluripotent stem cells involves transferring the pluripotent stem cells into an appropriate differentiation medium which promotes differentiation of the pluripotent stem cells into specific cell types. For instance, pluripotent stem cells can be differentiated into neural stem cells by culturing in a neural induction medium (NI medium, Gibco) with the addition of specific proteins and small molecules such as basic FGF, SB431542, and CHIR99021.

In some embodiments of the present disclosure, the phthalide compound may be selected from the group consisting of n-butylidenephthalide, methylphthalide, 7-methylphthalide, ethylphthalide, n-propylidenephthalide, n-butylphthalide, 3-bromophthalide, 5-bromophthalide, 5-chlorophthalide, 6-chlorophthalide, 3,4-dichlorophthalide, tetrachlorophthalide, 3-hydroxy-3-trifluoromethylphthalide, 3-methyl-3-(1-naphthyl)phthalide, 3-(5-fluoro-1-naphthyl)phthalide, 4-amino-3-hydroxyphthalide, 5-carboxyphthalide, 5-cyanophthalide, 7-methoxyphthalide, 7-hydroxy-6-methoxyphthalide, 3-(1,2-dimethyl-3-indolyl)phthalide, phenolphthalein, ligustilide, and sedanolide. In other embodiments of the present disclosure, the phthalide compound may be n-butylidenephthalide, n-butyl phthalide, tetrachlorophthalide, or phenolphthalein. In an exemplary embodiment, n-butylidenephthalide is used to illustrate the effect of selective removal of undifferentiated stem cells.

In one embodiment of the present disclosure, the effective amount of the phthalide compound is administered during a differentiation period of the pluripotent stem cells to selectively remove undifferentiated pluripotent stem cells. The differentiation period may be the differentiation of pluripotent stem cells into oligopotent stem cells, during which the differentiated cells in the cell population may not yet include somatic cells, or the differentiation of oligopotent stem cells into somatic cells. In another embodiment of the present disclosure, the effective amount of the phthalide compound is administered after the differentiation of pluripotent stem cells into somatic cells to achieve selective removal of undifferentiated pluripotent stem cells. Those skilled in the art can easily determine the appropriate differentiation period of the pluripotent stem cells differentiating into unspecific oligopotent stem cells or somatic cells and a time point of completion of differentiation based on cell surface antigens known in the art.

In one embodiment of the present disclosure, the concentration of the effective amount of the phthalide compound is about 10 µM to 1000 µM. In other embodiments of the present disclosure, the concentration of the effective amount of the phthalide compound is about 10 µM to 800 µM, about 50 µM to 800 µM, about 50 µM to 750 µM, or about 50 µM to 500 µM. In some embodiments of the present disclosure, the concentration of the effective amount of the phthalide compound is about 50 µM, 100 µM, 150 µM, 200 µM, 250 µM, 300 µM, 350 µM, 400 µM, 450 µM, 500 µM, 550 µM, 600 µM, 650 µM, 700 µM, 750 µM, or 800 µM. The aforementioned values can be optionally selected as the maximum or minimum to derive a numerical range.

In one embodiment of the present disclosure, the effective amount of the phthalide compound is administered for approximately 1 to 6 days. In other embodiments of the present disclosure, the effective amount of the phthalide compound is administered for about 24 hours, 48 hours, 72 hours, 96 hours, 120 hours, or 144 hours; the aforementioned numerical endpoints can be selected as either the maximum or minimum value to derive a numerical range.

In another embodiment of the present disclosure, the differentiated stem cells are treated with 100 µM of the phthalide compound for 6 days to selectively remove undifferentiated stem cell.

The present disclosure also relates to an use of the phthalide compounds for the selective removal of undifferentiated pluripotent stem cells.

The present disclosure will be further illustrated with specific examples which should not be considered as limitation to the scope of the present disclosure.

### Materials and Methods

### Culture and Differentiation of Human Pluripotent Stem cells

1. Culture of iPSCs in Essential 8 Feeder-Free Culture Medium: Human iPSCs were cultured in Essential 8 medium (Gibco) in culture dishes treated with basement membrane matrix (Matrigel). Subculture was performed when the cells reached 70% to 80% confluence, approximately 3 to 5 days. During subculture, the cells were rinsed twice with PBS until the edges began to lift, followed by treatment with a cell dissociation regent (Accutase) for 1 to 5 minutes to round up most of the cells. The cell dissociation regent was then diluted with DMEM/F 12, and DMEM medium was added. The cells were scraped with even force by a cell scraper, dispersed into cell mass with appropriate size by mechanical force, and plated at an appropriate ratio (about 1:5 to 1:10) with 10 µM of Y27632 added.
2. Dopaminergic Neuron Adherent Differentiation of Human iPSCs (CHSF-DA Differentiation Method): Human iPSCs, cultured in feeder-free environment, were subcultured at a 1:10 ratio for dopaminergic neurons differentiation. For induction, a neural induction medium composed of DMEM/F 12 (2:1) and N2 supplement were used and dopaminergic precursor cell-inducing factors basic FGF (FGF-basic, as known as FGF-2 or bFGF) (10 mg/mL), SB431542 (2 µM), CHIR99021 (7.5 µM), SAG (1 µM), and LDN193189 (0.2 µM) were added in days 1 to 12 of the differentiation process. On day 13 of the differentiation process, the medium was switched to a neuron culture medium (Neurobasal medium and N2/B27 supplements), with SAG (0.5 µM) added from days 13 to 18. SAG was removed on day 18, and 100 µM of n-butylidenephthalide was added on days 23 to 28 for a total of 6 days. The cells were subsequently cultured in Neurobasal medium with N2/B27 supplements, the medium was changed every 2 days during the differentiation process with proteins and small molecules re-added.
3. Neural Differentiation of Human iPSCs Using the Serum-Free Embryoid Body (SFEB) Method: The differentiation of iPSCs into neural cells was performed in four steps. The first three steps involved SFEB suspension, in which suspended spheroid cells were cultured for 35 days, and the fourth step is a 3-day cell adhesion growth:
   Step 1: iPSCs were aggregated into suspended embryoid bodies (EBs), which is an initial step of differentiation. iPSCs were treated with 1 mg/mL Dispase II until the edges rolled up, then rinsed three times with PBS. The iPSCs were collected by a cell scraper and dispersed into cell mass with appropriate size by visual inspection of pipet. After suspended in Essential 6 medium containing RevitaCell, and the iPSCs were transferred to a sterile, non-adherent 6 cm² culture plate for a 2-day suspension culture, with daily medium changing.
   Step 2: The EBs were transferred to a 15 mL centrifuge tube for settlement at room temperature, and the EBs were cultured in suspension for 2 days by adding neural induction medium (NI medium, Gibco) after removal of the supernatant. Basic FGF (10 ng/mL), SB431542 (10 µM), and CHIR99021 (3 µM) were added, with daily observations under a microscope.
   Step 3: The neural induction medium in Step 2 was replaced with Neurobasal medium (NB medium, Gibco), and the cells from Step 2 were cultured in suspension with continuous addition of basic FGF (10 ng/mL). The medium was changed every two days in this stage, with a total culture period of 31 days.
   Step 4: The cells were attached to a culture plate coated with 1% basement membrane matrix or laminin/ornithine. The culture medium was Neurobasal medium with basic FGF (10 ng/mL). The cells grow outward from the center of the colony, and after 3 days of continued culture, the cells were ready for experimentation.
4. Neural Stem Cell Adhesion Differentiation of Human iPSCs (Neural Stem Cell Adhesion Differentiation Method): Human iPSCs, cultured in a feeder-free environment, were subcultured at a 1:10 ratio for neural stem cell differentiation. The differentiation was induced by using a neural induction medium composed of DMEM/F 12 (2:1) and N2 supplement, with the addition of basic FGF (10 mg/mL), SB431542 (2 µM), and CHIR99021 (3 µM). The medium was changed every two days with proteins and small molecules re-added. Culture time for this neural stem cell adhesion differentiation is up to 7 days.

### Administration of n-Butylidenephthalide (N-BP)

a. Drug Efficacy and Concentration Testing: n-Butylidenephthalide was diluted to different concentrations and administered to cells at final concentrations ranging from 50 to 750 µM. The types of administered cells include cultured human iPSCs, human iPSCs aggregated into SFEBs for one day, neural stem cells on day 10 of SFEB differentiation, neurons on day 25 of SFEB differentiation, and cells on day 5 of neural differentiation by the adhesion. The concentrations and treatment durations were as described in the Examples.
b. Application of n-Butylidenephthalide in Dopaminergic Neuron Preparation: 100 µM of n-Butylidenephthalide was added from days 23 to 28 during the CHSF-DA differentiation process for a total of 6 days.

### Cell Characterization and Functional Assessment

a. Immunofluorescence Staining Method: Chamber slides were surface-treated with 1% basement membrane matrix for 3 to 4 hours, and the slides were set aside after the matrix was removed. Cells were enzymatically dissociated into smaller clumps and seeded onto the chamber slides. After a few days of culture until the cells adhered and extended outward to present a rosette-like neural tube morphology, identification and staining for neural stem cell and various neuronal precursor cell markers are performed. The culture medium was removed before immunofluorescence staining, and the cells were gently washed 2 to 3 times with PBS at room temperature or 37°C. Adding 200 µL of 4% paraformaldehyde to the cell, the cells were fixed after 20 minutes at room temperature. After removing the 4% paraformaldehyde, the cells were gently washed three times PBS, each for 5 minutes, and PBS was then removed. Adding 200 µL of 99% methanol or 0.1 to 0.3% Triton for 5 to 10 minutes at 4°C to perforate the cell membranes. After removal and evaporation of methanol or Triton, the cells were gently washed three times with PBS, 5 minutes each time. Adding 5% horse serum at room temperature for 1 hour to mask the cells, and then adding primary antibodies prepared in 3% horse serum (the concentration thereof is prepared based on the required concentration of the antibody) after removal of 5% horse serum. After overnight conjugation of the primary antibody, the primary antibody was removed, and the cells were washed three times with PBST (PBS with Tween 20) for 5 minutes each. Adding the secondary antibody with an action time of 1 hour at room temperature in the dark, and the secondary antibody is prepared in PBS at a concentration of 1:500. The secondary antibody was removed, and the cells were gently washed three times with PBST for 5 minutes each time. Adding 200 µL of DAPI (1 µg/mL) to the cells for 10 minutes at room temperature in the dark to perform nuclear staining. After removal of DAPI, the cells were washed twice with PBST for 5 minutes each time. After removing the PBST, PBS was added to keep the cells moist. The chamber slides were removed, and the cells were mounted with a long coverslip and mounting medium. The slides were stored in the dark at 4°C for subsequent analysis under a fluorescence microscope.
b. Whole-Cell Patch-Clamp Neuroelectrophysiological Testing:

### Buffer Solution:

1. Artificial cerebrospinal fluid (aCSF): NaCl 127 mM/KCl 3 mM/NaHCO₃ 26 mM/NaH₂PO₄ 1.25 mM/CaCl₂ 2 mM/MgSO₄ 1 mM/D-Glucose 10 mM, pH was adjusted to 7.45.
2. Pipette Solution: The pipette solution consists of K gluconate 140 mM/NaCl 10 mM/EGTA 0.5 mM/HEPES 10 mM/ATP-Mg 3 mM/and GTP 0.4 mM, pH was adjusted to 7.3.

Recording: Differentiated neurons derived from iPSCs were attached to coverslips and placed in a recording chamber containing artificial cerebrospinal fluid (95% O₂ + 5% CO₂). The electrodes used were made from 1.5 mm/1.0 mm inner diameter capillary glass (World Precision Instruments PG52151-4), drawn using a Sutter P-97 puller (Sutter Instrument, Novato, CA) and polished with an MF-830 microforge (Narishige, Tokyo, Japan). The electrodes were filled with pipette solution. Neuronal electrophysiology was recorded using an Axoclamp 200B (Axon Instruments, Union City, CA). Membrane currents were recorded in voltage clamp mode, while action potentials were recorded in current clamp mode. The following steps were used for cell stimulation and recording:
1. The cell potential was maintained at -60 mV, and incrementally increased by 10 mV every 400 ms from -80 mV to +40 mV to detect sodium and potassium ion currents.
2. The current was increased from -60 pA to +120 pA in 20 pA increments, and action potentials were recorded.

c. Quantitative Analysis of Dopamine Secretion Using ELISA Sandwich Method: Differentiated neurons were dissociated using Accutase, and subcultured at a density of 4×10⁵ cells per well in a 6-well plate. Following stimulation with KCl, the culture medium was collected every 24 or 48 hours, centrifuged at 1000 rpm for five minutes, and the supernatant was rapidly stored at -80°C. The culture medium (or concentrated culture medium) was then subjected to ELISA quantification (Beckman Coulter).

d. Calcium Ion Imaging Analysis: Cells were seeded onto 10 mm diameter coverslips coated with Geltrex and cultured in NB medium supplemented with RevitaCell and Compound E for 3 days. A 1 µM solution of Fluo-4 was prepared in physiological buffer. The coverslips were incubated in the Fluo-4 solution at 37°C for 40 minutes, followed by incubation in physiological buffer at 37°C for 20 minutes. The coverslips were then transferred to a calcium imaging chamber for perfusion imaging. The cells were perfused with physiological buffer for 30 seconds, then switched to 60 mM KCl perfusion for one minute, followed by physiological buffer perfusion for five minutes. Subsequently, the cells were perfused with 1 mM L-glutamic acid for one minute, followed by physiological buffer perfusion for five minutes. Images were captured using a Nikon ECLIPSE Ti2-E microscope and analyzed with NIS-Elements AR software.

### Parkinson's Disease Assessment

Animal transplantation experiment: A stereotaxic instrument was used to inject 6-OHDA into the striatum of rats to destroy dopamine neurons in the substantia nigra, thereby inducing a physiological response similar to human Parkinson's disease. One month after 6-OHDA injection and dopamine neuron damage induction, the rats were injected subcutaneously with methamphetamine hydrochloride (2 mg/kg). The number of rotations was recorded using a rotameter for a total of 60 minutes; rats with more than 300 rotations per hour were considered to have successfully induced dopamine neuron damage. Behavioral tests were conducted three days before surgery, and on day 30 post-induction, pluripotent stem cells were differentiated into precursor dopamine neurons and injected into the anterior substantia nigra (striatum) at the following coordinates: bregma: A, +1.0; L, -3.0; V, -5.0 and -4.0; TB, 0; with 2×10⁵ cells/µL, 2 µL/site. Behavioral testing was performed at 1, 2, 3, 4, 5, and 6 months using a rotometer. At week 26, the animals were sacrificed, and tissues were collected for subsequent immunofluorescence (IF) and immunohistochemistry (IHC) analysis.

### Example 1

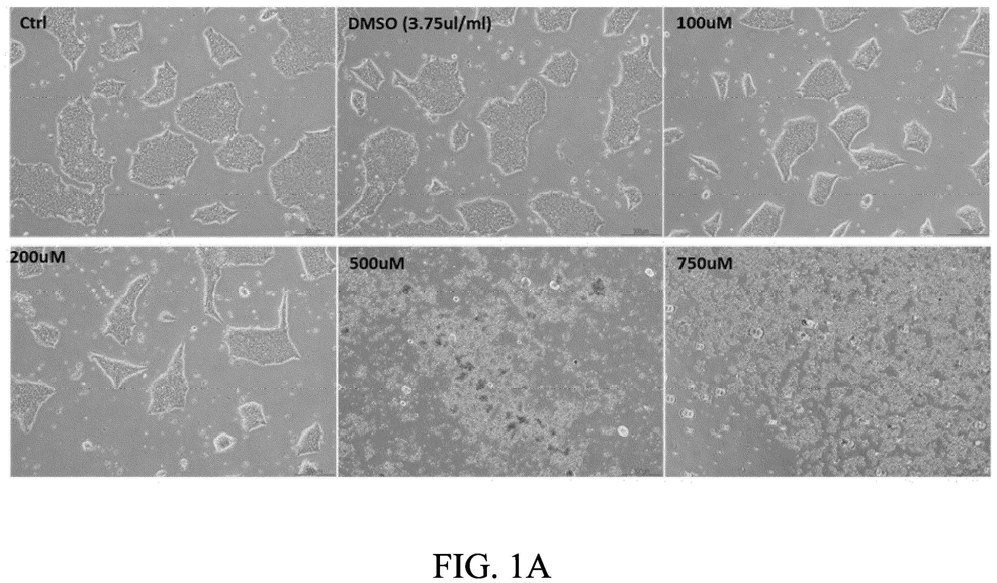

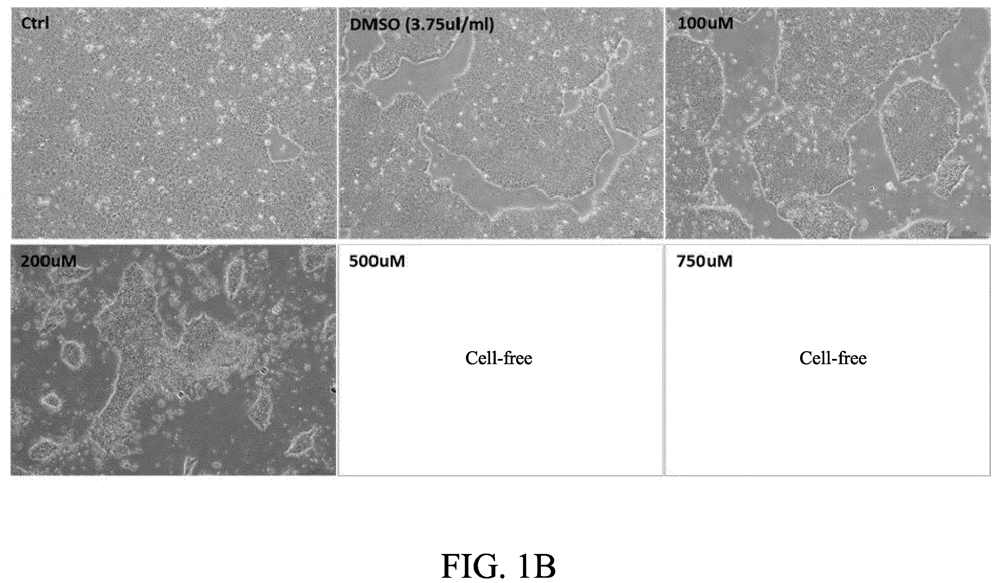

Human induced pluripotent stem cells (iPSCs) were collected and cultured in Essential 8 medium without a feeder layer. After 24 hours of treatment with different concentrations of n-butylidenephthalide (100 µM, 200 µM, 500 µM, and 750 µM), the cell morphology was shown in Figure 1A. It was observed that the number of human iPSCs treated with 100 µM n-butylidenephthalide was significantly lower than that in the control group (Ctrl) and the DMSO control group, and human iPSCs treated with concentrations above 500 µM n-butylidenephthalide did not adhere or survive. After 48 hours of treatment with different concentrations of n-butylidenephthalide, the cell morphology was shown in Figure 1B. It was observed that the number of human iPSCs treated with 100 µM n-butylidenephthalide was significantly lower than that in the control group (Ctrl) and the DMSO control group, and cells in the 200 µM treatment group showed obvious floating and cell death. No cells survived in the human iPSCs treated with concentrations above 500 µM n-butylidenephthalide.

### Example 2

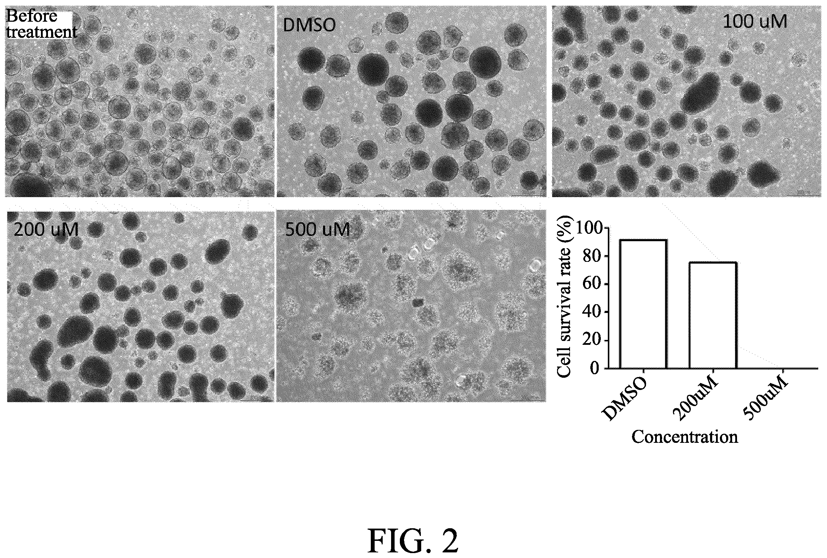

Cultured and collected iPSCs were aggregated into spheres using the SFEB suspension method and suspended in culture medium to simulate the three-dimensional structure of general pluripotent cell differentiation and transplantation. After only one day of suspension differentiation, the cells aggregated into structurally complete spheres (embryoid bodies). After 24 hours of treatment with different concentrations of n-butylidenephthalide (100 µM, 200 µM, and 500 µM), it was observed in Figure 2 that the suspended spheres treated with 500 µM n-butylidenephthalide had all dispersed and died. Trypan blue staining revealed a decrease in viable cells at 200 µM, and no viable cells were observed at 500 µM.

### Example 3

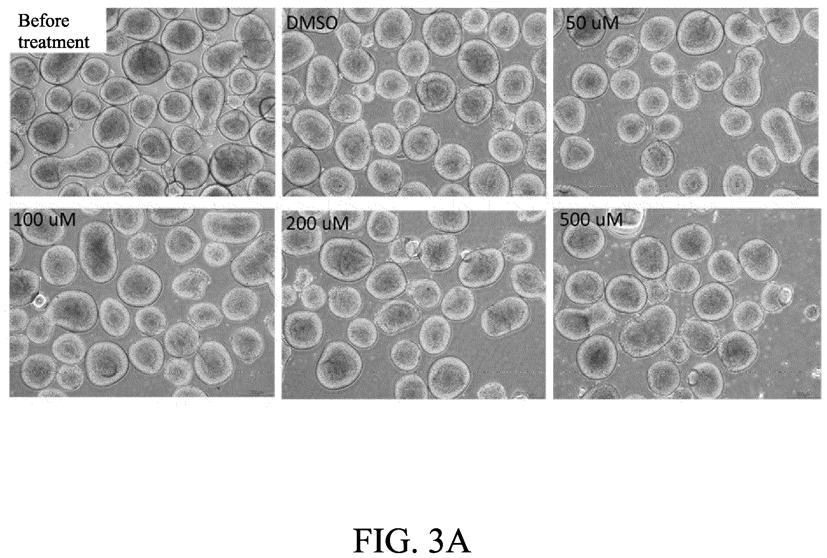

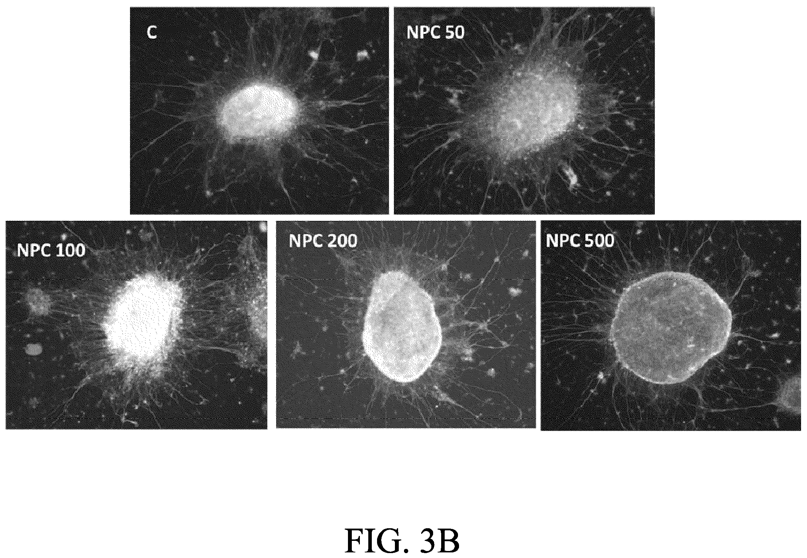

Collected human iPSCs were aggregated into spheres using the SFEB suspension method and differentiated into neural stem cells. During the differentiation process, the cells were cultured in a neural induction medium composed of DMEM/F12 (2:1) with the addition of basic FGF, SB431542, and CHIR99021 as neural induction factors. Starting on day 10 of suspension differentiation, different concentrations of n-butylidenephthalide (50 µM, 100 µM, 200 µM, and 500 µM) were added for 120 hours. As shown in Figure 3A, no cell death or morphological changes were observed in the suspended spheres. To confirm whether the neural stem cells treated with n-butylidenephthalide still retained the ability to differentiate into mature neurons, the treated spheres were cultured following the SFEB neural differentiation method until day 38 of differentiation. Immunofluorescence staining was used to analyze the expression of neuron-specific transcription protein NeuN and neurofilament (NF). The experimental results, shown in Figure 3B, indicated that neural stem cells treated with n-butylidenephthalide were able to differentiate normally into mature neurons, expressing NeuN (green fluorescence) and NF (red fluorescence), and displayed a typical nerve fiber morphology.

### Example 4

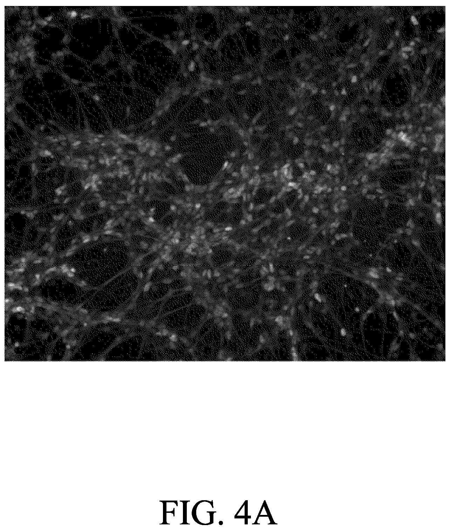

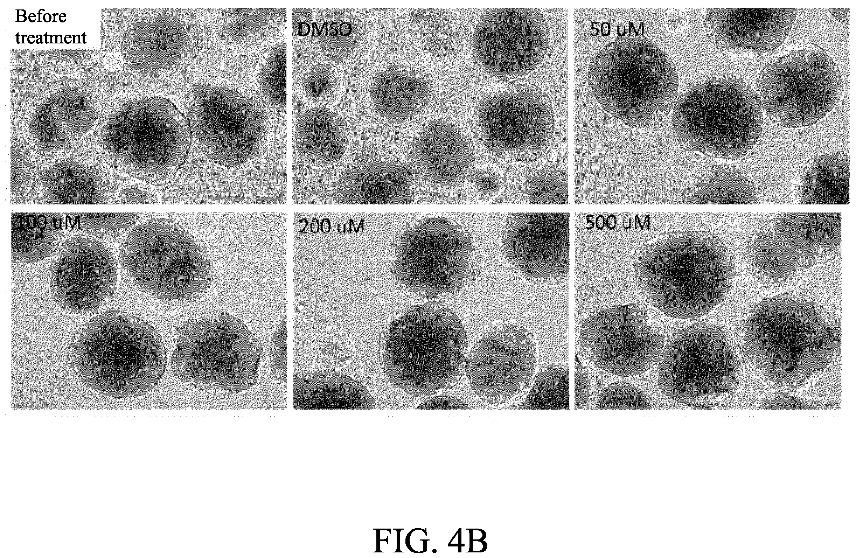

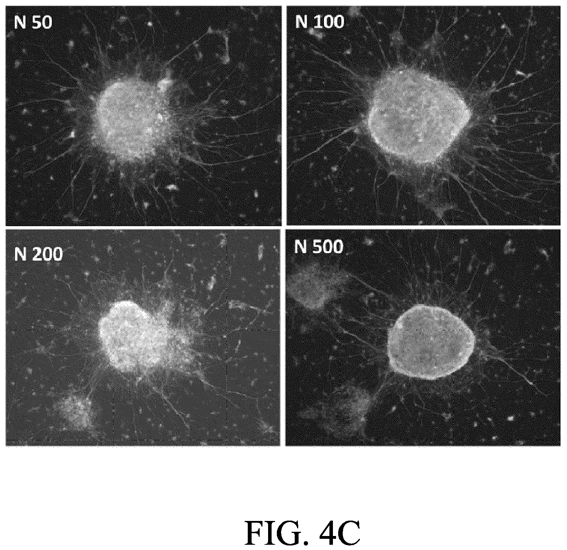

To confirm whether n-butylidenephthalide was harmful to differentiated somatic cells, neuronal cell spheroids differentiated up to day 25 using the SFEB suspension method were collected. As shown in Figure 4A, cells were cultured according to the SFEB suspension method until day 22, then transferred early to step four of the SFEB neural differentiation method for attachment culture for three days. Immunofluorescence staining revealed the expression of mature neuron marker proteins NeuN (green fluorescence) and NF (red fluorescence), indicating that the cells differentiated to day 25 by the SFEB suspension method were indeed neuronal cells, i.e., differentiated neuronal cells. These neuronal cell spheroids, differentiated to day 25, were then cultured for 120 hours with varying concentrations of n-butylidenephthalide (50 µM, 100 µM, 200 µM, and 500 µM). As shown in Figure 4B, no damage to the spheroids or cell death was observed. Furthermore, as illustrated in Figure 4C, the mature neurons continued to differentiate until day 38 using the SFEB neural differentiation method, showing NeuN (green fluorescence) and NF (red fluorescence) expression and exhibiting typical nerve fiber morphology.

### Example 5

Three sets of cells were prepared: one set of cells cultured without n-butylidenephthalide treatment (Ctrl), another set cultured with 500 µM n-butylidenephthalide for 120 hours on day 10 of differentiation (NPC-BP500), and a third set cultured with 500 µM n-butylidenephthalide for 120 hours on day 25 of differentiation (Neuron-BP500). These cells were initially cultured using the SFEB neural differentiation method until day 35 of differentiation, at which point they were attached and cultured for an additional three days. The nerve cells, differentiated using the SFEB neural differentiation method, were tested using a calcium ion imaging system to observe changes in calcium ion flow across the cell membrane after stimulation with KCl and glutamic acid. As shown in Figures 5A and 5B, all three groups of neurons (Ctrl, NPC-BP500, Neuron-BP500) responded significantly to potassium ions and neurotransmitters, with no statistically significant differences between them, indicating that the nerve cells treated with 500 µM n-butylidenephthalide retained normal neuroelectrophysiological function.

### Example 6

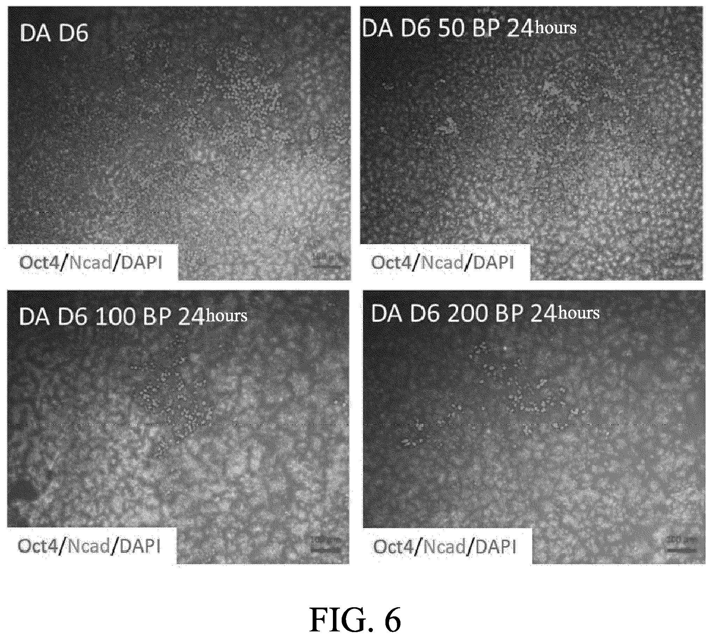

The collected human iPSCs were differentiated into neurons using the neural stem cell attachment differentiation method. During differentiation, a neural induction culture medium composed of DMEM/F12 (2:1) and N2 supplement was used, along with the neural induction factors basic FGF, SB431542, and CHIR99021. As shown in Figure 6, on day five of differentiation, approximately half of the cells expressed the pluripotent stem cell-specific protein Oct-4, while the other half expressed the neural stem cell protein N-cadherin. After treatment with 50 µM, 100 µM, and 200 µM n-butylidenephthalide for 24 hours, immunofluorescence staining analysis revealed that the proportion of undifferentiated pluripotent stem cells expressing Oct-4 decreased significantly as the concentration of n-butylidenephthalide increased.

### Example 7

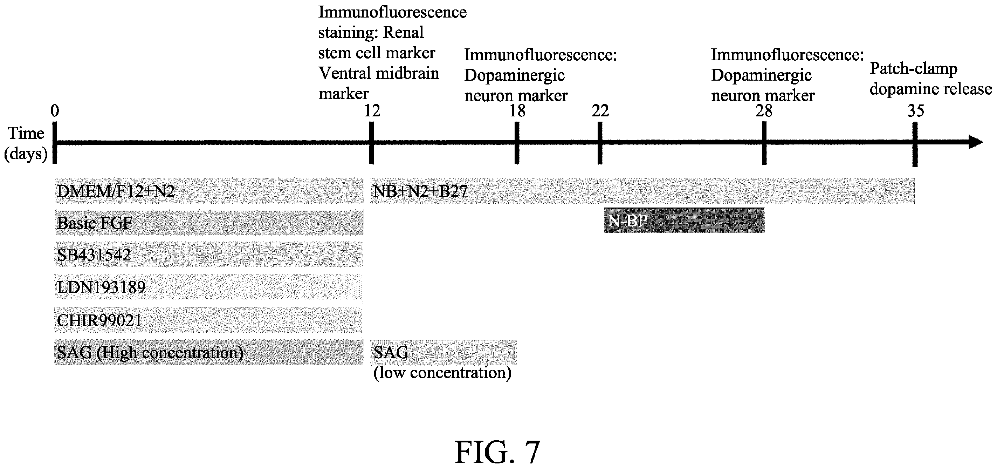

The dopamine neuron attachment differentiation process (CHSF-DA differentiation method) was applied to the collected human iPSCs. The test procedure for identifying differentiating dopamine neurons was outlined in Figure 7. The process was briefly described as follows: on day 12 of differentiation, immunofluorescence staining was used to identify neural stem cell-specific proteins Sox-1 and N-cadherin, dopamine precursor cell-specific proteins FOXA2, Lmx1A, and Corin, to verify whether the neural stem cells on day 12 of differentiation were dopamine neuron precursor cells. On days 18 to 28 of differentiation, mature dopamine neuron proteins TH and Nurr1, and ventral midbrain dopamine neuron protein Aldh1A1 were identified. On day 35 of differentiation, the dopamine content in the neuron culture medium was tested using the ELISA antibody method to confirm the dopamine secretion ability of the dopamine neurons, and single-cell patch-clamp was used to assess their neuroelectrophysiological function. The results were shown in Figures 8A to 8M.

Figures 8A to 8C show the changes in cell morphology during differentiation. On day four of differentiation (Figure 8A), the cells still exhibited the typical iPSC morphology. By day 11 (Figure 8B), the cells had transitioned to a neural stem cell-like rosette arrangement. By day 17 (Figure 8C), numerous nerve fiber-like structures were observed. Figures 8D to 8F demonstrate that on day 12 of differentiation, the majority of cells simultaneously expressed both neural stem cell and dopamine precursor cell-specific proteins. Between days 18 and 28 of differentiation (Figures 8G to 8J), various dopamine neuron-specific proteins were expressed. On day 35 of differentiation, dopamine neurons underwent functional assessment. Figure 8K shows that dopamine was only secreted into the culture medium following KCl stimulation. Figure 8L indicates that typical inward and outward feedback currents were observed on the neuron surface in response to different voltage stimulations. Figure 8M shows that feedback potential differences were generated by the neurons following stimulation with different currents. The above results demonstrate that the dopamine neurons generated through this differentiation process exhibited normal morphology, specific protein expression, neuroelectrophysiological responses, and dopamine secretion functions, indicating that the addition of n-butylidenephthalide did not affect the expression of specific proteins or the neurological function of dopamine neurons.

### Example 8

A rat model of Parkinson's disease was established by destroying unilateral midbrain dopamine neurons using 6-OHDA. After successfully inducing Parkinson's disease, the rats were evenly divided into two groups based on the average number of unilateral rotations. Brain surgery was performed six weeks post-6-OHDA, with one group (preBP-DA, 21 rats) receiving dopamine neurons differentiated for 28 days and treated with n-butylidenephthalide for six days, while the other group (blank injection, eight rats) received an equal volume of injection solution without cells. Post-transplantation, Cyclosporine A (15 mg/kg) was administered daily to suppress immune rejection. The 21 preBP-DA rats were divided into a specimen group and a behavior test group. The specimen group (12 rats) was sacrificed at 2, 4, and 8 weeks post-cell transplantation, with brain specimens collected for tissue staining to determine the differentiation and survival of the transplanted cells. The remaining nine rats (behavior test group) underwent rotameter behavioral tests at 4, 8, 12, 16, 20, and 24 weeks post-cell transplantation, with natural survival rates recorded up to 26 weeks. All nine rats in the behavior test group were sacrificed 26 weeks post-cell transplantation, and brain specimens were collected to analyze the survival and maturation of the transplanted cells.

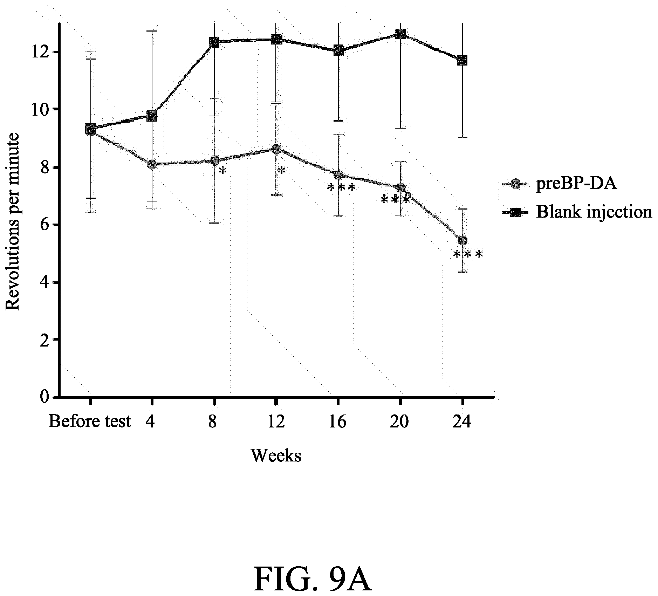

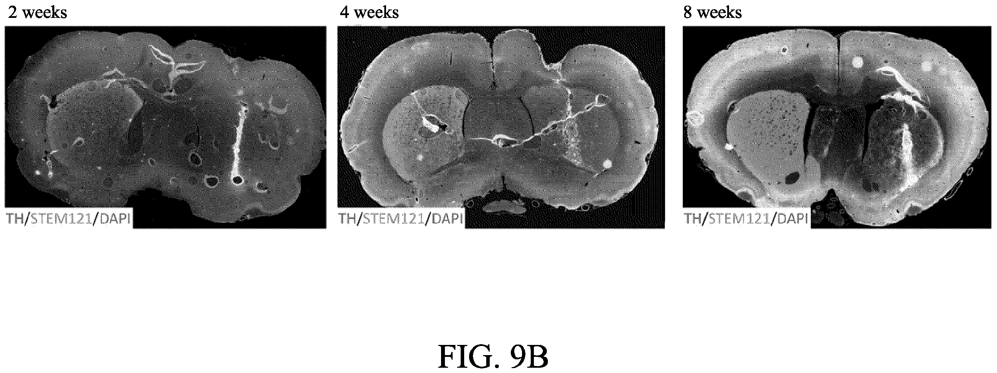

Figure 9B shows immunofluorescence staining of rat brain sections at 2, 4, and 8 weeks post-transplantation, with TH (red, marking dopamine neurons), STEM121 (green, marking human cells), and DAPI (blue, marking cell nuclei). The right side of the brain in Figure 9B represents the side where the rat's original dopamine neurons were removed by 6-OHDA and human cells were transplanted. The left side represents the healthy, untreated brain side. Comparison of the left and right brain sides indicated that dopamine neurons treated with n-butylidenephthalide survived for two weeks post-transplantation in the rat brain, proliferated, and began to mature into dopamine neurons between weeks 2 and 4, and matured into dopamine neurons extensively between weeks 4 and 8, spreading throughout the striatum. Additionally, Figure 9A demonstrates the improvement in motor abilities of Parkinson's disease rats that received the aforementioned dopamine neuron transplants.

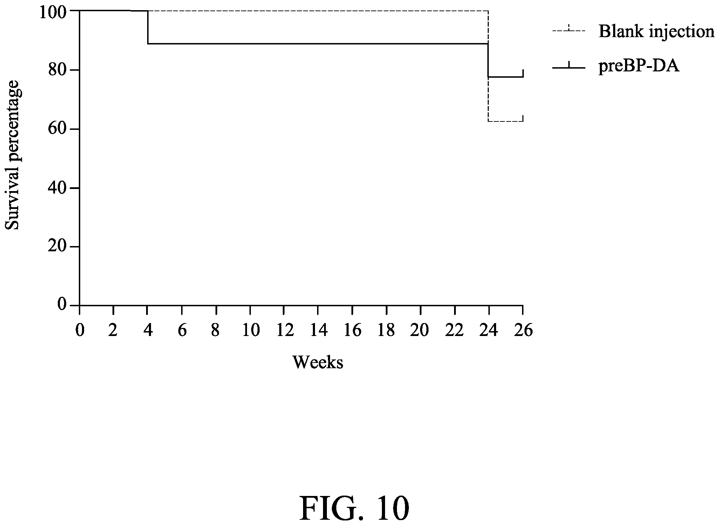

Figure 10 summarizes the survival rates by calculating the number of surviving rats/total rats (%) every four weeks. It shows that the survival rate of rats with transplanted dopamine neurons treated with n-butylidenephthalide was not lower than that of the blank injection control group after 24 weeks, and Table 1 indicates that no tumors or teratoma-like tissues were observed in the brain sections of cell-transplanted animals. This suggests that dopamine neurons treated with n-butylidenephthalide pose no tumor risk or other observable harmful effects post-transplantation.

The combined results from Figures 9A, 9B, and 10 indicate that following brain transplantation of cells treated with n-butylidenephthalide, there were no tumor risks or detectable adverse effects on the animals, and the treatment was effective.

**Table 1**

| Brain Tissue Collection Time (Weeks Post-Cell Transplantation)) | Number of Rats | Presence of Tumor or Teratoma-like Tissue at Cell Transplantation Site |
|---|---|---|
| 2 weeks | 4 | 0 |
| 4 weeks | 4 | 0 |
| 8 weeks | 4 | 0 |
| 26 weeks | 9 | 0 |

Although the present disclosure has been described in detail through some specific embodiments above, various modifications and changes may be made to the illustrated embodiments by those skilled in the art without substantially departing from the teachings and advantages of the present disclosure. Therefore, such modifications and changes should still be considered within the scope of the present disclosure as defined by the appended claims.

## Claims

1. A method for selectively removing undifferentiated pluripotent stem cells, comprising:
collecting pluripotent stem cells;
inducing differentiation of the collected pluripotent stem cells to obtain a cell population comprising differentiated cells and undifferentiated pluripotent stem cells; and
administering an effective amount of a phthalide compound to the cell population to selectively remove the undifferentiated pluripotent stem cells in the cell population.

2. The method of claim 1, wherein the phthalide compound is at least one selected from the group consisting of n-butylidenephthalide, methyl phthalide, 7-methyl phthalide, ethyl phthalide, n-propenyl phthalide, n-butyl phthalide, 3-bromophthalide, 5-bromophthalide, 5-chlorophthalide, 6-chlorophthalide, 3,4-dichlorophthalide, tetrachlorophthalide, 3 -hydroxy-3 -trifluoromethylphthalide, 3 -methyl-3 -(1 - naphthyl)phthalide, 3-(5-fluoro-1-naphthyl)phthalide, 4-amino-3-hydroxyphthalide, 5-carboxyphthalide, 5-cyanophthalide, 7-methoxyphthalide, 7-hydroxy-6-methoxyphthalide, 3-(1,2-dimethyl-3-indolyl)phthalide, phenolphthalein, ligustilide, and cerdanolide.

3. The method of claim 1, wherein the differentiated cells include at least one selected from the group consisting of oligopotent stem cells, unipotent stem cells, and somatic cells.

4. The method of claim 1, wherein the differentiated cells in the cell population do not include somatic cells.

5. The method of claim 1, wherein the effective amount of the phthalide compound is administered after a presence of somatic cells in the cell population.

6. The method of claim 1, wherein a concentration of the effective amount of the phthalide compound is from 10 µM to 1000 µM.

7. The method of claim 6, wherein the concentration of the effective amount of the phthalide compound is from 50 µM to 800 µM.

8. The method of claim 1, wherein the effective amount of the phthalide compound is administered for 1 to 6 days.

9. The method of claim 1, wherein the pluripotent stem cells are embryonic stem cells or induced pluripotent stem cells.
